# EUROPEAN PATENT SPECIFICATION

(11) **EP 2 952 220 B1**
(45) Date of publication and mention of the grant of the patent: **13.11.2019**
(21) Application number: 14745502.6
(22) Date of filing: 29.01.2014
(51) Int. Cl.: A61M 25/00, A61M 25/06, A61M 5/162, A61M 5/50

(54) **PERIPHERAL INTRAVENOUS CATHETER WITH BELLOWS-TYPE PASSIVE SAFETY SYSTEM IVCBTS**
PERIPHERER INTRAVENÖSER KATHETER MIT EINEM PASSIVEN SICHERHEITSSYSTEM MIT FALTENBÄLGEN
CATHÉTER INTRAVEINEUX PÉRIPHÉRIQUE POSSÉDANT UN SYSTÈME DE SÉCURITÉ PASSIVE DE TYPE SOUFFLET CIVSTF

(30) Priority: 30.01.2013 MX 2013000051 U
(43) Date of publication of application: 09.12.2015
(73) Proprietor: Equipos Médicos Vizcarra, S.A., Emiliano Zapata, Morelos 62765 (MX)
(72) Inventor: ARELLANO CABRERA, José Antonio, Morelos C.P 62765 (MX); RODRIGUEZ LELIS, José María, Morelos C.P 62765 (MX); LUCAS JIMENEZ, Maria Teresa, Morelos C.P 62765 (MX); CHALITA VIZCARRA, Alfredo, Morelos C.P 62765 (MX)
(74) Representative: Temiño Ceniceros, Ignacio
(86) International application number: PCT/MX2014/000031
(87) International publication number: WO 2014/119986

(56) References cited:
- EP-A2- 0 747 083
- EP-A2- 1 430 921
- WO-A1-01/47582
- WO-A1-93/08865
- WO-A2-2006/032995
- JP-A- 2005 152 282
- US-A- 4 160 450
- US-A- 5 053 014
- US-A- 5 695 474
- US-A- 5 695 474
- US-A1- 2007 270 754
- US-A1- 2011 054 403
- US-A1- 2011 319 827
- US-B1- 6 375 640

## Description

### FIELD OF THE INVENTION

The present invention is related to the hospital instruments manufacturing industry. More specifically it is related to the industry which manufactures instruments for venous puncturing and catheterization. Even more specifically, it is related to the industry which manufactures instruments for puncturing and canalization, and a protection system to prevent accidental puncture wounds to the user and others by the needle which is contaminated with the patient's fluids.

### BACKGROUND OF THE INVENTION

Nowadays, devices for placing catheters are common. When a catheter is placed in a patient for intravenous administering of a liquid, a disposable need is used which passes through the catheter to puncture a vein and allow a catheter to enter. There are essentially two kinds of catheter introducing assemblies for introducing intravenous catheters into a patient. The first kind is a catheter placed inside a puncturing needle which is used to puncture the skin and place the catheter into the circulatory system of the patient; the needle is removed, leaving the catheter in the patient's circulatory system. The second kind of catheter introducing assembly consists of a disposable needle that passes through the catheter to puncture the vein and allows a catheter to be inserted. Once it has been checked that the assembly has been placed in the vein, using a flashback of the blood in the needle, the needle, contaminated with the patient's fluids or blood is removed, leaving the intravenous catheter in place for connection with an intravenous bag or bottle, or a stopper for later use.

The main problem in using this kind of device occurs during and following the catheterization procedure; the technician in charge of the procedure has to handle the needle contaminated with blood, which is still sharp and there is a possibility that the technician or others may accidentally suffer a puncture wound.

There is worldwide epidemiological evidence that the main concern about using sharp instruments and their disposal as infectious waste in hospitals is the transmission of the AIDS virus and more frequently, Hepatitis B and C virus. This is due to wounds caused by needles contaminated with human blood. The former has created a growing need for catheter producers, in preventing accidental wounds with needles by medical personnel or other employees, who handle hospital waste, wash the clothing or other residues which the used needles may contain. As a result, the new products are designed to incorporate special covers for the needle or mechanisms for retracting the needle inside a protective chamber. Such devices are described, for example, in the U.S. patents 4.747.831, 4.828.548, 5.129.884, 5.501.675, 5.817.058, 5.989.220, 7.771.394 y 7.740.615. Many of the devices described in these patents have numerous pieces which substantially increase manufacturing costs and are sometimes difficult to handle. Other devices require both hands to operate or are prone to untimely activation of the safety system while it is being transported, stored and handled. Besides, other devices only encapsulate the sharp end of the needle which allows blood or fluid stuck to the rest of the body of the needle to contaminate users and others who are in contact with the needle.

As may be inferred from the text, there are various qualities which an intravenous catheterization device should have. Among these are: (a) a device which does not require specialized training to use; (b) reduces the possibility of multiple punctures; (c) a device with a passive protection system in order to prevent accidental punctures; and (d) to be easy to handle. These are the characteristics claimed in the present invention.

In a catheterization device with rapid and painless puncturing, a means for protecting the needle has been presented, in order to protect the user and prevent accidental puncture wounds with the tip of the needle. However, in a catheter without a rapid and painless puncturing system, there has not been any system presented for protecting the needle as indicated in the present application.

As an example of the kind of protection mentioned in the preceding paragraph, may be found in EP2604307A (State of the art according to Art. 54(3)EPC).

Another example of intravenous catheters with safety system IVCBTS is US5695474 that discloses a specific configuration of catheter having a safety system that consist of a flexible tube with an affixing body and a mounting body at both ends of the flexible tube, but cannot have a connectable flashback chamber with filter.

### OBJECTIVE OF THE INVENTION

The main objective of the invention is to achieve a simple device which possesses a passive protection system which protects against accidental puncture wounds and at the same time is easy to activate.

Another objective is to reduce the need for experience, of the person using the device, in using it and reducing the risk of contamination to the user with the patient's bodily fluids.

All of these objectives and advantages will become apparent through the reading and drawings which accompany it with purely illustrative, non-limiting ends and which form an integral part of the present description.

### BRIEF DESCRIPTION OF THE INVENTION

The invention resides in the catheter of claim 1.

The present invention refers to a system for introducing an intravenous catheter with a cannula with retractable needle in a manual billows mechanism in order to prevent its re-use, accidental puncture wounds and to assure its safe disposal.

The present invention refers to a double system which presents, in one of its components, a compact, easy-to-use system for performing the placing of a peripheral intravenous catheter in a patient's circulatory system. The second component is a manual safety mechanism which after carrying out the puncturing, withdraws the contaminated cannula into a protective billows chamber which prevents later contact with users or other persons, thus preventing accidental puncture wounds and the transmission of diseases.

Moreover, the characteristics of the passive retractable system assure that there is no contact at all with the cannula after placing the catheter in the patient and it allows for placing it at a safe distance within the protection chamber of the bellows type security system.

The peripheral intravenous catheter assembly with a bellows-type passive safety system IVCBTS of the present invention is made up of a hollow hub with two opposing longitudinal openings. On one of the ends of the catheter hub, there is coaxially aligned and anchored, a catheter tube; while the other open end opposite allows the passage of an introducing needle which remains within the catheter tube presenting a Luer connection, once the catheter is in the patient's vein, to connect to the infusion lines for medication or IV solution.

The hollow hub connects, on the side opposite the placement of the catheter, to an affixing body, which has an opening for the introducing needle to pass through. This first affixing body, in the first stage of operation of IVCBTS is used by medical personnel, who will place the catheter in the patient, to hold the device and carry out the catheterization procedure. In the second stage of operation of IVCBTS it blocks the sharp point of the introducing needle in order to avoid accidental puncture wounds.

Attached to the other end of the affixing body is a flexible bellows tube, which will be used to house the introducing needle when the IVCBTS safety system is activated. Initially the bellows is compressed, while the other end of the bellows butts up against the mounting body which acts as a base for mounting the introducing needle. With this configuration the introducing needle passes through the flexible bellows tube, from the affixing body, crosses the hub of the catheter and is placed inside the catheter. All of these components of the aforementioned configuration make up the IVCBTS in its puncturing aspect.

Also attached to one end of the mounting body is a hollow translucent cylinder or filter, which allows the technician responsible for the catheterization procedure to see the flashback of blood when the far introducing tip of the needle is inserted into the patient's vein.

Once the catheterization procedure has been carried out and the introducing needle along with the catheter are in the patient's vein, the technician responsible for carrying out the procedure, without moving the affixing body, pulls the mounting body away from the catheterization area. This process withdraws the introducing needle from the patient's vein, leaving the catheter in place. The bellow is also deployed in order to cover the part of the cannula which was in contact with the bodily fluids including blood and the introducing needle is encapsulated so as to prevent possible accidental puncture wounds and the flow of contaminated fluids from the patient.

### BRIEF DESCRIPTION OF THE DRAWINGS

Figure 1 shows an exploded view of the peripheral intravenous catheter with a bellows-type passive safety system IVCBTS showing the parts that make it up.
Figure 2 is an isometric view of the peripheral intravenous catheter with a bellows-type passive safety system IVCBTS showing how the connection is made between the puncturing system and the safety system with the peripheral intravenous catheter, besides a detailed view of the configuration and connecting elements.
Figure 3 shows an isometric view of the peripheral intravenous catheter with a passive IVCBTS bellows safety system.
Figure 4 shows a lateral cross section view of the peripheral intravenous catheter with a bellows-type passive safety system IVCBTS in its puncturing stage.
Figure 5 shows an isometric view of the peripheral intravenous catheter with a bellows-type passive safety system IVCBTS in the safety stage in order to prevent accidental puncture wounds and contact with the patient's contaminated fluids.
Figure 6 shows a lateral cross section view of the peripheral intravenous catheter with a bellows-type passive safety system IVCBTS in its safety stage and also details of the components which block the point of the puncturing cannula.
Figure 7 shows an isometric view of the peripheral intravenous catheter with a bellows-type passive safety system IVCBTS in its safety stage and the uncoupling of the peripheral intravenous catheter.
Figure 8 shows a lateral cross section view of the peripheral intravenous catheter with a bellows-type passive safety system IVCBTS in its safety stage and uncoupling of the peripheral intravenous catheter.
Figure 9 shows an isometric view of the primary casing of the peripheral intravenous catheter with a passive IVCBTS billows safety system.
Figure 10 shows an isometric view of the protective tube with the cap and the uncoupling of the peripheral intravenous catheter with a bellows-type passive safety system IVCBTS ready to be used.

### DETAILED DESCRIPTION OF THE INVENTION

We shall use the drawings of the preferred aspect of the present invention to support our detailed description.

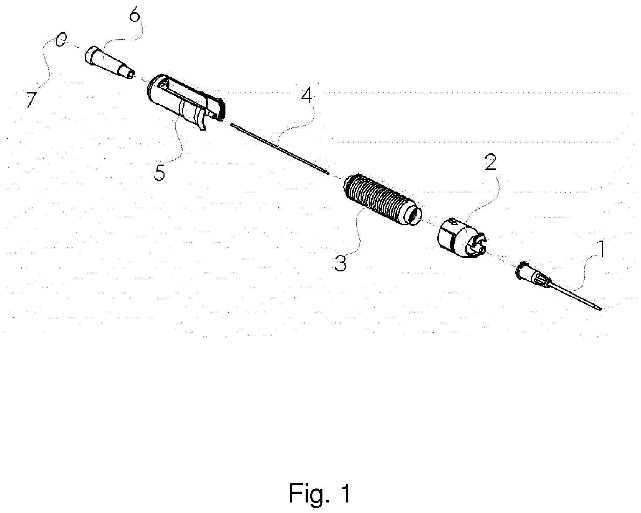

An exploded view of the seven elements which make up the peripheral intravenous catheter with a bellows-type passive safety system IVCBTS is shown in Figure 1. As can be seen in this Figure 1, the parts which make up the IVCBTS are: (1) catheter, (2) affixing body, (3) flexible bellows tube, (4) puncturing cannula, (5) mounting body, (6) flashback chamber and filter and (7) filter paper.

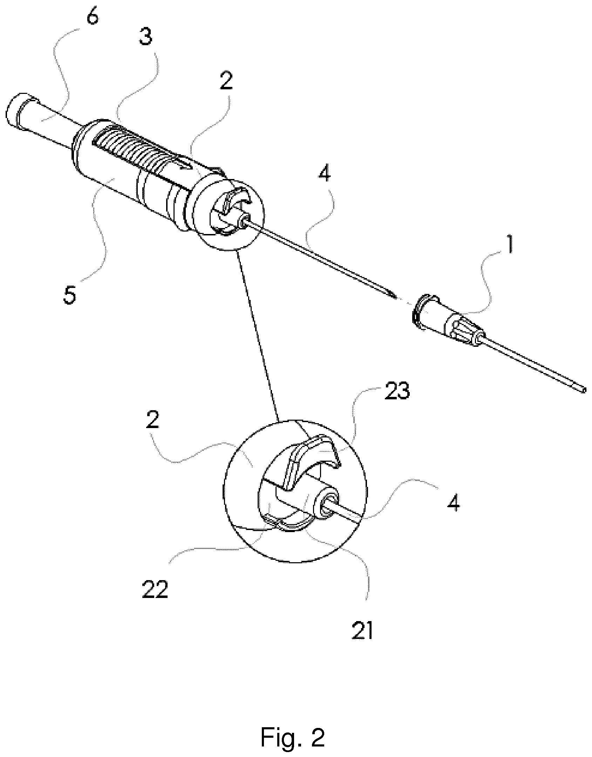

The isometric view of the closed peripheral intravenous catheter and the puncturing and safety system in the stage of connection is shown in Figure 2. The affixing body (2) meets, on the distal end, the flexible billows tube (3) and at the same time meets the mounting body (5) which holds the puncturing cannula (4) which passes through the flexible bellows tube (3) and the affixing body (2). While on the other end of the mounting body (5) the flashback camber (6) and filter are attached: all of these components form the puncturing and safety system. The puncturing and safety system is attached to the peripheral intravenous catheter by means of a mounting post (21) on the affixing body (2), through which the puncturing cannula (4) passes concentrically to enter the catheter (1) and this in turn rests on the flat surface (22) of the affixing body (2).

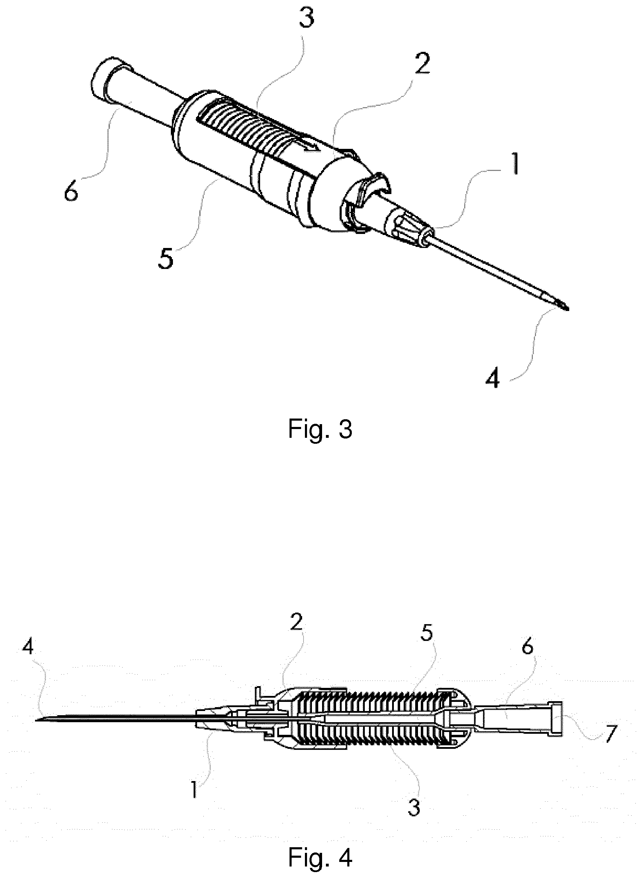

Once the safety and puncturing system are connected to the peripheral intravenous catheter, the peripheral intravenous catheter with a bellows-type passive safety system IVCBTS is formed, a symmetric view of which may be seen in Figure 3. The IVCBTS is made up of the mounting body (5) which is connected to the flexible bellows tube (3) and the affixing body (2) to which the catheter (3) is attached. The puncturing cannula (4) is attached to the mounting body (5) and passes concentrically through the flexible billows tube (3) and the affixing body (2) to cross it and remain inside the catheter (1).

In the puncturing stage of the IVCBTS as shown in a cross-section in Figure 4, the puncturing cannula (4) joins the mounting body, not shown in this figure, at one of its ends while on the other end is attached the flashback chamber (6) and filter forming the channel through which the blood signal passes when the introducing cannula penetrates the patient's vein and also to prevent overflow of blood from the channel of the puncturing system a filter paper (7) is placed on the free end of the filter and flashback chamber (6).

Attached to the affixing chamber (5) is the flexible billows tube (3) in a compressed state and it covers part of the puncturing cannula (4), while on the other end of the flexible bellows tube (3) an affixing body is attached (2). The puncturing cannula (4) passes through the affixing body (2) crossing it and remaining housed inside the catheter (1) which is attached to the affixing body (2).

During the catheterization procedure the medical personnel in charge of the procedure holds the IVCBTS of the affixing body (2) and introduces the puncturing cannula (2) along with the catheter (1) into the circulatory system of the patient. By means of the flashback chamber (6) and filter the technician may confirm the correct placement of the peripheral intravenous catheter in the patient's circulatory system and may proceed to withdraw the puncturing cannula (4), activating the passive IVCBTS safety system.

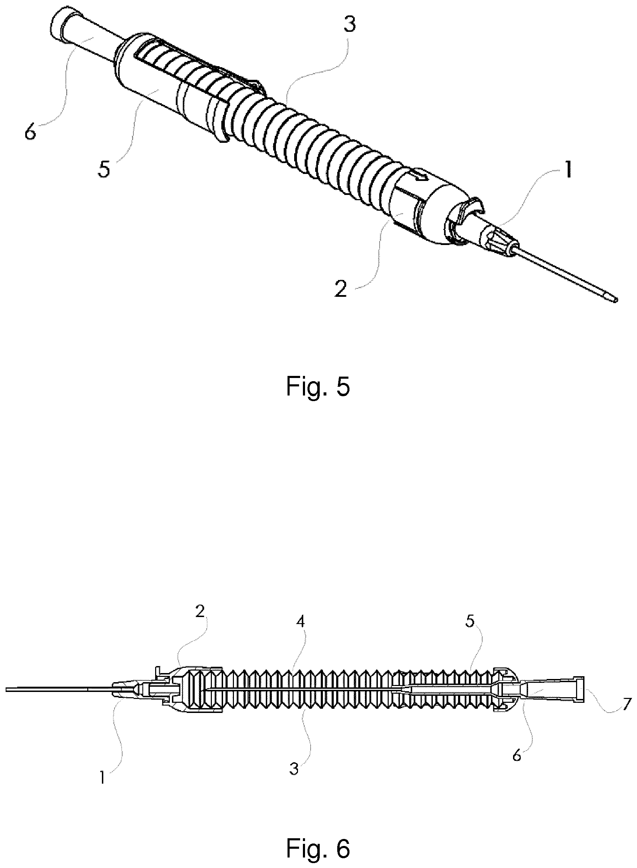

The IVCBTS in its safety stage is shown in an isometric view in Figure 5. Once the catheter (1) is correctly placed into the patient's circulatory system, the technician in charge of the procedure, holding the IVCBTS in place with the affixing body (2) pulls the mounting body (5) away from catheterization area and this causes the flexible bellows tube (3) to expand and the puncturing cannula (4) is withdrawn from inside the catheter (1) and the patient's vein.

In Figure 6 we can see how the puncturing cannula (4) has remained completely encapsulated within the flexible bellows tube (3) which has stretched in length. The flexible billows tube is connected to the affixing body (2) and the mounting body (5) on opposite ends. When the technician pulls the mounting body (5), the flexible bellows tube (3) expands and at the same time withdraws the puncturing needle which is at the end of the retraction within the flexible billows tube, thus preventing splashing with the patient's contaminated blood or fluids within the used puncturing cannula (4). The puncturing cannula passes through the opening in the affixing body (21) retained by the hatch of the affixing body (24) which makes the passage of the puncturing cannula (4) through the opening of the affixing chamber (21) difficult and disabling the sharp ends of the needle of the puncturing cannula (4).

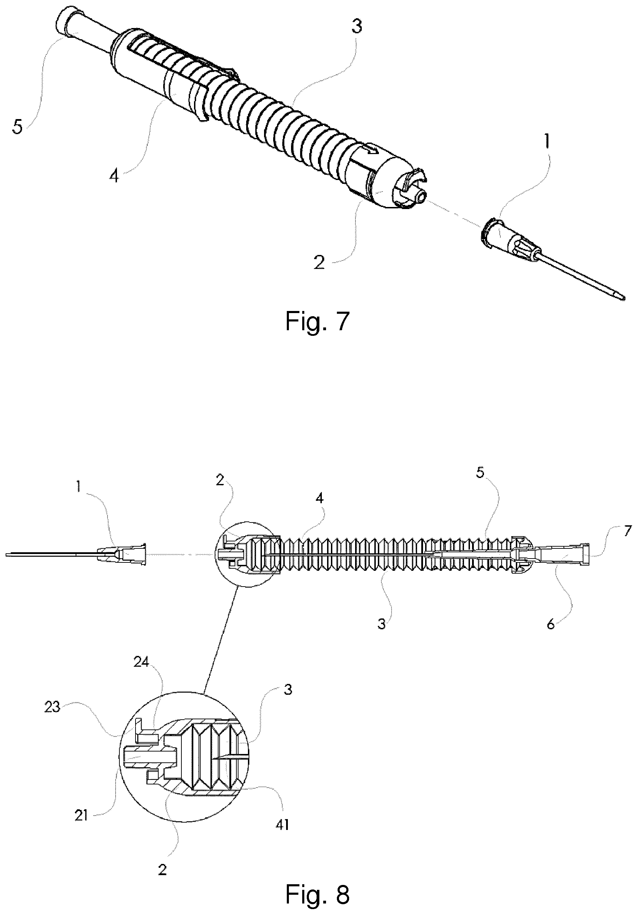

Once the flexible bellows tube (3) has expanded and the puncturing cannula (4) is encapsulated and the sharp ends of the needle disabled by the affixing body (2), the technician in charge proceeds to disconnect the previously activated safety system from the peripheral intravenous catheter as shown in Figure 7. The peripheral intravenous catheter is firmly affixed to the area of catheterization and at this point the intravenous infusion line for medications may be attached.

Once the peripheral intravenous catheter is properly inserted into the patient's circulatory system, the puncturing cannula (4), contaminated with the patient's blood and fluids, may be disposed of. The puncturing cannula remains encapsulated inside the flexible bellows tubing (3) and the sharp end of the needle blocked by the affixing body (11) so as to prevent possible accidental puncture wounds to medical personnel and their contamination with the blood which adheres to the used puncturing cannula (4).

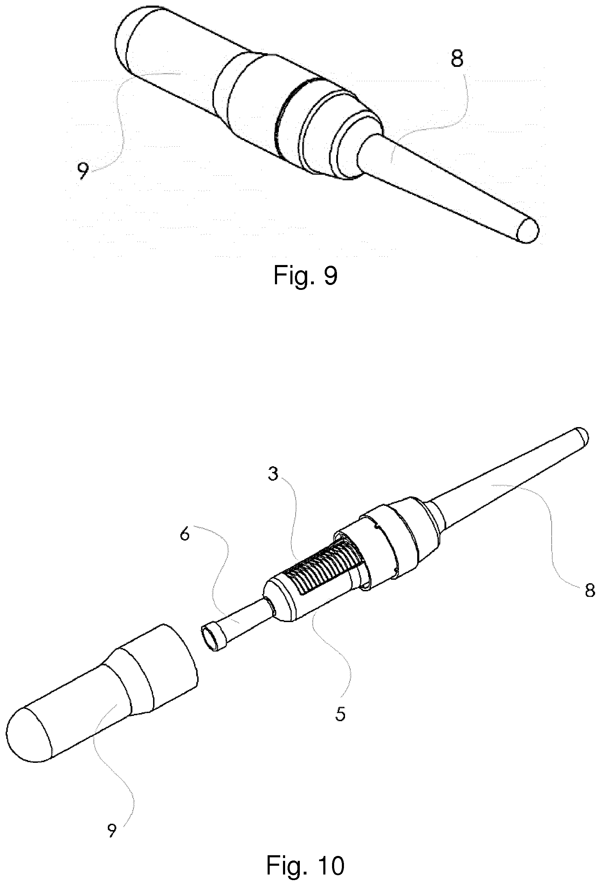

The primary cover of the peripheral intravenous catheter with a bellows-type passive safety system IVCBTS is shown in Figure 9. The primary cover consists of a protective tube (8) and a cap (9). The configuration shown in Figure 9 presents the protective tube (8) and the cap (9) which together form a rigid capsule in which the IVCBTS is found with the characteristics that it does not permit the passage of sterilizing material and prevents the passage of microorganisms.

Once the patient has been prepared for catheterization, the technician removes the cap (8) by pulling away from the protective tube (9) so that the rear part of the IVCBTS can be seen but with the puncturing cannula (4) still covered by the rigid barrier of the protective tube (8) in order to prevent accidental puncture wounds as shown in Figure 10. Once the technician is ready to carry out catheterization, he/she removes the protective tube from the IVCBTS, leaving the puncturing cannula (4) and the catheter (1) uncovered in order to carry out the puncturing procedure.

The invention has been sufficiently described so that a person with knowledge of the field can reproduce and obtain the results we mention for the present invention. However, anyone with knowledge of the field of the present invention is capable of making modifications not described in the present application, and if, for the application of these modifications in the determined structure or manufacturing process it is necessary to use the material claimed in the following claims, said structure should be considered within the scope of the invention.

## Claims

1. A peripheral intravenous catheter with bellows-type passive safety system IVCBTS, which includes a safety system for preventing accidental puncture wounds, consisting of: a flexible bellows tube (3) with an affixing body (2) on one of the longitudinal ends and a mounting body (5) on the other end, a puncturing cannula and a catheter holder, said bellows tube (3) being connected on its longitudinal ends to an affixing body (2) and a mounting body (5) so that it forms a capsule into which the puncturing cannula (4) is retracted and housed once it has been used, wherein said affixing body (2) consists of a means for connection for the catheter holder, the catheter being **characterized in that** a flashback chamber (6) and filter is firmly connected at the proximal end to the distal end of the cannula and includes at least one external peripheral step which allows for the partial introduction in the mounting body (5), and the affixing body (2) and the catheter holder each have half of the connecting means on their respective ends, and wherein the affixing body (2) has on its proximal face a projection proximally with two interruptions on opposite peripheral sides which act to latch a peripheral tab of the catheter holder.

2. The peripheral intravenous catheter with bellows-type passive safety system IVCBTS, as described in Claim 1, **characterized in that** a filter paper (7) is placed on the free end of the filter and flashback chamber (6).

3. The peripheral intravenous catheter with bellows-type passive safety system IVCBTS, as described in Claim 1, **characterized in that** the filter is a hollow translucent cylinder.

## Patentansprüche

1. Peripherer intravenöser Katheter mit passivem Sicherheitssystem vom Balgtyp IVCBTS, der ein Sicherheitssystem zum Verhindern versehentlicher Stichwunden aufweist, bestehend aus: einen Balgschlauch (3) mit einem Befestigungskörper (2) an einem der Längsenden und einem Montagekörper (5) am anderen Ende, eine Punktionskanüle und einen Katheterhalter, wobei der Balgschlauch (3) an seinen Längsenden mit einem Befestigungskörper (2) und einem Montagekörper (5) verbunden ist, so dass er eine Kapsel bildet, in welche die Punktionskanüle (4) zurückgezogen und aufgenommen wird, nachdem sie verwendet wurde, wobei der Befestigungskörper (2) aus einem Mittel zum Verbinden für den Katheterhalter besteht, wobei der Katheter **dadurch gekennzeichnet ist, dass** eine Rückschlagkammer (6) und ein Filter am proximalen Ende fest mit dem distalen Ende der Kanüle verbunden sind und mindestens einen externen peripheren Schritt aufweist, der das teilweise Einführen in den Montagekörper (5) ermöglicht, und der Befestigungskörper (2) und der Katheterhalter jeweils die Hälfte der Verbindungsmittel an ihren jeweiligen Enden aufweisen, und wobei der Befestigungskörper (2) an seiner proximalen Fläche einen Vorsprung proximal mit zwei Unterbrechungen auf gegenüberliegenden peripheren Seiten aufweist, die zum Einrasten in eine periphere Lasche des Katheterhalters fungieren.

2. Peripherer intravenöser Katheter mit passivem Sicherheitssystem vom Balgtyp IVCBTS nach Anspruch 1, **dadurch gekennzeichnet, dass** ein Filterpapier (7) an dem freien Ende des Filters und der Rückschlagkammer (6) angeordnet ist.

3. Peripherer intravenöser Katheter mit passivem Sicherheitssystem vom Balgtyp IVCBTS nach Anspruch 1, **dadurch gekennzeichnet, dass** der Filter ein hohler lichtdurchlässiger Zylinder ist.

## Revendications

1. Cathéter intraveineux périphérique à système de sécurité passive de type soufflet CIVSTS, qui comporte un système de sécurité pour prévenir les blessures accidentelles par piqûre, consistant en : un tube à soufflet flexible (3) avec un corps de fixation (2) à l'une des extrémités longitudinales et un corps de montage (5) à l'autre extrémité, une canule de ponction et un porte-cathéter, ledit tube à soufflet (3) étant relié par ses extrémités longitudinales à un corps de fixation (2) et à un corps de montage (5) de manière à former une capsule dans laquelle la canule de perforation (4) est rétractée et logée une fois qu'elle a été utilisée, dans lequel ledit corps de fixation (2) est constitué d'un moyen de raccordement pour le porte-cathéter, le cathéter étant **caractérisé en ce qu'**une chambre de retour (6) et filtre est raccordée fermement à l'extrémité distale de la canule et comporte au moins un échelon périphérique externe qui permet l'introduction partielle dans le corps de montage (5), et le corps de fixation (2) et le porte-cathéter ont chacun la moitié des moyens de raccordement à leurs extrémités respectives, et dans lequel le corps de fixation (2) a sur sa face proximale une saillie proximale avec deux interruptions sur les côtés périphériques opposés qui agissent pour verrouiller une languette périphérique du porte-cathéter.

2. Cathéter intraveineux périphérique avec système de sécurité passif de type soufflet CIVSTS, tel que décrit dans la revendication 1, **caractérisé en ce qu'**un papier filtre (7) est placé sur l'extrémité libre du filtre et chambre de retour (6).

3. Cathéter intraveineux périphérique avec système de sécurité passif de type soufflet CIVSTS, tel que décrit dans la revendication 1, **caractérisé en ce que** le filtre est un cylindre translucide creux.
